# EUROPEAN PATENT SPECIFICATION

(11) **EP 2 271 612 B1**
(45) Date of publication and mention of the grant of the patent: **10.08.2016**
(21) Application number: 09726618.3
(22) Date of filing: 31.03.2009
(51) Int. Cl.: C07C 211/42, A61K 9/14, A61K 31/205, A61P 25/16

(54) **RASAGILINE MESYLATE PARTICLES AND PROCESS FOR THE PREPARATION THEREOF**
RASAGILINMESYLATPARTIKEL UND VERFAHREN ZU DEREN HERSTELLUNG
PARTICULES DE MÉSYLATE DE RASAGILINE ET LEUR PROCÉDÉ DE PRÉPARATION

(30) Priority: 31.03.2008 IN CH07882008
(43) Date of publication of application: 12.01.2011
(73) Proprietor: Actavis Group PTC EHF, 220 Hafnarfjordur (IS)
(72) Inventor: PATIL, Nilesh, Sudhir, Jalgaon 425 001 Maharashtra (IN); PAGIRE, Haushabhau, Shivaji, Maharashtra (IN); NEELA, Praveen, Kumar, Hyderabad 500 036 Andhra Pradesh (IN); PRADHAN, Nitin, Sharadchandra, Thane (West) 400 601 Maharashtra (IN); VALGEIRSSON, Jon, IS-220 Hafnarfjörour (IS)
(74) Representative: Cole, William Gwyn
(86) International application number: PCT/IB2009/005643
(87) International publication number: WO 2009/122301

(56) References cited:
- WO-A-95/11016
- WO-A-2006/091657
- DATABASE WPI Section Ch, Week 200882 Thomson Scientific, London, GB; Class GFD, AN 2008-O02376 [82] XP002544646 & CN 101 260 048 A (SUZHOU CHIREACH BIOMEDICAL TEC) 10 September 2008 (2008-09-10)

## Description

### FIELD OF THE DISCLOSURE

Disclosed herein is rasagiline mesylate having a particle size which is suitable for homogeneous distribution of the drug substance in a tablet blend.

### BACKGROUND

U.S. Patent No. 5,532,415 (hereinafter referred to as the '415 patent) discloses R(+)-N-propargyl-1-aminoindan (rasagiline) and its pharmaceutically acceptable salts, processes for their preparation, pharmaceutical compositions, and methods of use thereof. Rasagiline has been shown to be a selective inhibitor of the B-form of the enzyme monoamine oxidase (MAO-B), useful in treating Parkinson's disease and various other conditions by inhibition of MAO in the brain. Rasagiline has the molecular formula of C₁₂H₁₃N, molecular weight of 171.24, and a structural formula of:

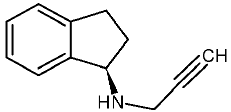

The mesylate salt of rasagiline is a selective and potent irreversible inhibitor of the B-form of the enzyme monoamine oxidase sold by Teva under the brand name Azilect®. Methods of preparing rasagiline mesylate are described in U.S. Patent No. 5,532,415.

PCT Publication No. WO 95/11016 and U.S. Patent No. 6,126,968 disclose pharmaceutical compositions comprising rasagiline salts.

PCT Publication No. WO 2006/091657 discloses solid pharmaceutical formulations of rasagiline comprising an amount of the mixture of particles of a pharmaceutically acceptable salt of rasagiline, wherein more than 90% of the total amount by volume of rasagiline salt particles have a size of less than 250 microns.

Rasagiline mesylate is a white to off-white powder, freely soluble in water or ethanol and sparingly soluble in isopropanol.

The solid state physical properties of an active pharmaceutical ingredient (API), such as rasagiline mesylate, can be very important in formulating a drug substance and can have profound effects on the ease and reproducibility of formulation. Particle size, for example, may affect the flowability and mixability of a drug substance. In cases, where the active ingredient has good flow properties, tablets can be prepared by direct compression of the ingredients. However, in many cases the particle size of the active substance is small, the active substance is cohesive or has poor flow properties. Small particles are also filtered and washed more slowly during isolation processes, and thus may increase the time and expense of manufacturing a drug formulation.

There is a need for rasagiline mesylate particles having a large particle size which is suitable for homogeneous distribution of the drug substance in a tablet blend, and has good flow properties, better dissolution and solubility properties to obtain formulations with greater bioavailability.

### SUMMARY

The present invention relates to rasagiline mesylate having a 90 volume-percent of the particles (D₉₀) with a size of 600 microns to 1500 microns, wherein the rasagiline mesylate has a total purity of 99% to 99.99% as measured by HPLC.

The invention also relates to a process for preparing rasagiline mesylate having a D₉₀ particle size of 600 microns to 1500 microns, comprising:
(a) providing a solution of rasagiline mesylate in a solvent medium comprising an ester solvent and an alcohol solvent;
(b) subjecting the solution from step-(a) to gradual cooling to produce a cooled solution;
(c) optionally, seeding the solution obtained in step-(b); and
(d) crystallizing rasagiline mesylate having a D₉₀ particle size of 600 microns to 1500 microns from the cooled solution,
wherein the rasagiline mesylate obtained has a total purity of 99% to 99.99% as measured by HPLC.

In another aspect, the rasagiline mesylate obtained by the process disclosed herein has a D₉₀ particle size of 650 microns to 1500 microns, specifically 700 microns to 1500 microns, more specifically 800 microns to 1500 microns, still more specifically 1200 microns to 1500 microns, and most specifically about microns to 1500 microns.

Described herein is a process for controlling the particle size of rasagiline mesylate, comprising:
a) providing solid particles of rasagiline mesylate having a D₉₀ particle size of 600 microns to 1500 microns; and
b) milling the rasagiline mesylate of step-(a) to obtain rasagiline mesylate particles having a particle size which is suitable for homogeneous distribution of the drug substance in a tablet blend, in particular 90 volume-percent of the particles (D₉₀) have a size of 255 microns to 1400 microns.

The rasagiline mesylate disclosed herein for use in the pharmaceutical compositions may have a 90 volume-percent of the particles (D₉₀) with a size of 255 microns to 1500 microns, specifically 260 microns to 1400 microns, more specifically 270 microns to 800 microns, still more specifically 280 microns to 600 microns, and most specifically 300 microns to 500 microns.

Also described herein is a pharmaceutical composition comprising rasagiline mesylate having a D₉₀ particle size of 255 microns to 1500 microns, and one or more pharmaceutically acceptable excipients.

Also described herein is a pharmaceutical composition comprising rasagiline mesylate having a D₉₀ particle size of 255 microns to 1500 microns made by the process disclosed herein, and one or more pharmaceutically acceptable excipients.

Also described is a process for preparing a pharmaceutical formulation comprising combining rasagiline mesylate having a D₉₀ particle size of 255 microns to 1500 microns with one or more pharmaceutically acceptable excipients.

Also described herein is a substantially pure rasagiline mesylate has a D₉₀ particle size of 255 microns to 1500 microns, having a relatively low content of one or more organic volatile impurities.

### DETAILED DESCRIPTION

Extensive experimentation has been carried out by the present inventors to produce rasagiline mesylate having a desired particle size which is suitable for homogeneous distribution of the drug substance in a tablet blend and has good flow properties. As a result, it has been found that rasagiline mesylate particles having a large particle size can be prepared by providing a solution of rasagiline mesylate in a solvent medium comprising an ester solvent and an alcohol solvent, and crystallizing rasagiline mesylate from the solution.

According to one aspect, provided herein is rasagiline mesylate having a 90 volume-percent of the particles (D₉₀) with a size of 600 microns to 1500 microns.

Rasagiline mesylate having a large particle size, disclosed herein, can be filtered and dried easily. Large particle size rasagiline mesylate allows the preparation of a final product containing less residual solvent and water than that containing small particles.

According to another aspect, there is provided a process for the preparation of rasagiline mesylate having a 90 volume-percent of the particles (D₉₀) with a size of 600 microns to 1500 microns, comprising:
a) providing a solution of rasagiline mesylate in a solvent medium comprising an ester solvent and an alcoholic solvent;
b) subjecting the solution from step-(a) to gradual cooling to produce a cooled solution;
c) optionally, seeding the cooled solution obtained in step-(b); and
d) crystallizing rasagiline mesylate particles having a D₉₀ particle size of 600 microns to 1500 microns from the cooled solution.

The particle size of the rasagiline mesylate obtained by the process disclosed herein allows the dissolution rate of the rasagiline mesylate to be controlled. Processing rasagiline mesylate to bring the particle size within a particular range can also enhance manufacturing capability, allowing the preparation of pharmaceutical compositions that exhibit an improved bioavailability of rasagiline mesylate. Large particle size rasagiline mesylate obtained by the process disclosed herein has good flow properties thus well-suited for pharmaceutical formulations.

Exemplary alcohol solvents used in step-(a) include, but are not limited to, C₁ to C₈ straight or branched chain alcohol solvents such as methanol, ethanol, propanol, isopropanol, n-butanol, tert-butanol, amyl alcohol, isoamyl alcohol, hexanol, and mixtures thereof. Specific alcohol solvents are methanol, ethanol, isopropanol, and mixtures thereof, and more specifically methanol. Exemplary ester solvents include, but are not limited to, ethyl acetate, isopropyl acetate, n-butyl acetate, tert-butyl acetate, and the like and mixtures thereof. A specific ester solvent is ethyl acetate.

In one embodiment, about 2 to 12 volumes, specifically, about 4 to 10 volumes of the ester solvent with respect to the alcohol solvent are used.

Step-(a) of providing a solution of rasagiline mesylate includes dissolving a form of rasagiline mesylate in the solvent medium, or obtaining an existing solution from a previous processing step.

In one embodiment, the rasagiline mesylate is dissolved in the solvent medium at a temperature of 70°C, to the reflux temperature of the solvent medium used, and more specifically at the reflux temperature of the solvent medium used.

As used herein, "reflux temperature" means the temperature at which the solvent or solvent system refluxes or boils at atmospheric pressure.

In another embodiment, the solution in step-(a) is prepared by suspending rasagiline mesylate in an ester solvent, stirring the suspension at reflux followed by slow addition of an alcoholic solvent to obtain the clear solution.

In further embodiment, the solution in step-(a) is prepared by admixing rasagiline base, methanesulfonic acid, and the solvent medium comprising an ester solvent and an alcoholic solvent to obtain a mixture; and heating the mixture to obtain a rasagiline mesylate solution. The heating is specifically carried out at a temperature of 70°C to the reflux temperature of the solvent used, and more specifically at the reflux temperature of the solvent used.

In yet another embodiment, the solution in step-(a) is prepared by admixing rasagiline base, methanesulfonic acid, and the solvent medium comprising an ester solvent and an alcoholic solvent to obtain a mixture; heating the mixture at a temperature of 70°C to the reflux temperature of the solvent used, specifically at the reflux of the solvent used; and adding an alcoholic solvent to obtain a rasagiline mesylate solution.

The gradual cooling of the solution in step-(b) is performed, for example, by slowly cooling the solution initially to a temperature of below 65°C, specifically at a temperature of 55°C to 65°C for at least 15 minutes, while slow stirring or with out stirring; maintaining the resulting solution at the same temperature for at least 15 minutes, specifically from 30 minutes to 3 hours; and further cooling the resulting solution to a temperature of below 50°C, specifically at a temperature of 40°C to 50°C for at least 15 minutes, while slow stirring or with out stirring; and maintaining the solution at the same temperature for at least 30 minutes, specifically for 3 hours to 15 hours.

In one embodiment, the crystallization in step-(d) is carried out by cooling the solution at a temperature of below 30°C for at least 15 minutes, specifically at 0°C to 30°C for 30 minutes to 20 hours, and more specifically at 15°C to 30°C for 1 hour to 15 hours.

The rasagiline mesylate solid obtained in step-(d) is recovered by techniques such as filtration, filtration under vacuum, decantation, and centrifugation, or a combination thereof. In one embodiment, the rasagiline mesylate solid can be recovered by filtration employing a filtration media of, for example, a silica gel or celite.

The process of the present invention can be performed on an industrial scale.

The pure rasagiline mesylate has a D₉₀ particle size of 600 microns to 1500 microns obtained by the above process may be further dried in, for example, a Vacuum Tray Dryer, Rotocon Vacuum Dryer, Vacuum Paddle Dryer or pilot plant Rota vapor, to further lower residual solvents. Drying is, for example, carried out under reduced pressure until the residual solvent content reduces to the desired amount such as an amount that is within the limits given by the International Conference on Harmonization of Technical Requirements for Registration of Pharmaceuticals for Human Use ("ICH") guidelines.

In one embodiment, the drying is carried out at atmospheric pressure or reduced pressures, such as below 267 mbar (200 mm Hg) or below 67 mbar (50 mm Hg) , at temperatures such as 35°C to 70°C. The drying is carried out for any desired time period that achieves the desired result, such as times 1 hour to 20 hours. Drying may be carried out for shorter or longer periods of time depending on the product specifications. Temperatures and pressures are chosen based on the volatility of the solvent being used and the foregoing should be considered as only a general guidance. Drying can be suitably carried out in a tray dryer, vacuum oven, air oven, or using a fluidized bed drier, spin flash dryer, flash dryer and the like. Drying equipment selection is well within the ordinary skill in the art.

The process disclosed herein can produce rasagiline mesylate in substantially pure form. The total purity of the rasagiline mesylate obtained by the process disclosed herein is of greater than 99%, specifically greater than 99.90%. and more specifically greater than 99.95% as measured by HPLC. For example, the purity of the rasagiline mesylate obtained by the process disclosed herein is 99% to 99.95%, or 99.5% to 99.99%.

According to another aspect, the substantially pure rasagiline mesylate obtained by the process disclosed herein has a relatively low content of one or more organic volatile impurities.

In one embodiment, the rasagiline mesylate obtained by the process disclosed herein comprises less than 200 parts per million (ppm) methanol, less than 500 ppm isopropyl alcohol, less than 100 ppm toluene, and less than 200 ppm ethyl acetate. Specifically, the rasagiline mesylate obtained by the process disclosed herein comprises less than 25 ppm methanol, less than 350 ppm isopropyl alcohol, less than 30 ppm toluene, and less than 30 ppm ethyl acetate.

In another embodiment, rasagiline mesylate obtained by the process disclosed herein has the overall level of organic volatile impurities less than 500 ppm, and more specifically less than 350 ppm.

Rasagiline mesylate particles obtained by the process disclosed herein have good flow properties and having a particle size which is suitable for homogeneous distribution of the drug substance in a tablet blend.

In one embodiment, the rasagiline mesylate obtained by the process disclosed herein has a D₉₀ particle size of 650 microns to 1500 microns, specifically 700 microns to 1500 microns, more specifically 800 microns to 1500 microns, still more specifically 1200 microns to 1500 microns, and most specifically 1400 microns to 1500 microns.

The particle sizes of the rasagiline mesylate, obtained by the process disclosed herein, can be further reduced by a mechanical process of reducing the size of particles which includes any one or more of cutting, chipping, crushing, milling, grinding, micronizing, trituration or other particle size reduction methods known in the art, to bring the rasagiline mesylate to the desired particle size range which is suitable for homogeneous distribution of the drug substance in a tablet blend.

The rasagiline mesylate having the large particle size, obtained by the process disclosed herein, specifically 600 microns to 1500 microns, can be milled to rasagiline mesylate having smaller particle size in a milling process that is adapted to the desired particle size. Thus, the milling process provides control over the obtained particle size of rasagiline mesylate. For example, milling can be performed by a cone mill, which operates by breaking particles with an impeller that revolves within a conical perforated screen.

Described herein is a process for controlling the particle size of rasagiline mesylate, comprising:
a) providing solid particles of rasagiline mesylate having a D₉₀ particle size of 600 microns to 1500 microns; and
b) milling the rasagiline mesylate of step-(a) to obtain rasagiline mesylate having a D₉₀ particle size of 255 microns to 1400 microns.

There is also described a process for producing rasagiline mesylate has a D₉₀ particle size of 255 microns to 1400 microns, comprising:
a) providing a solution of rasagiline mesylate in a solvent medium comprising an ester solvent and an alcoholic solvent;
b) subjecting the solution from step-(a) to gradual cooling to produce a cooled solution;
c) optionally, seeding the cooled solution obtained in step-(b);
d) crystallizing rasagiline mesylate having a D₉₀ particle size of 600 microns to about 1500 microns from the cooled solution; and
e) milling the crystalline rasagiline mesylate obtained in step-(d) to obtain the rasagiline mesylate having a D₉₀ particle size of 255 microns to 1400 microns.

Further encompassed herein is the use of substantially pure rasagiline mesylate obtained by the processes disclosed herein for the manufacture of a pharmaceutical composition.

A specific pharmaceutical composition of substantially pure rasagiline mesylate is selected from a solid dosage form and an oral suspension.

The rasagiline mesylate disclosed herein for use in the pharmaceutical compositions may have a 90 volume-percent of the particles (D₉₀) having a size of 255 microns to 1500 microns, specifically 260 microns to 1400 microns, more specifically 270 microns to 800 microns, still more specifically 280 microns to 600 microns, and most specifically 300 microns to 500 microns.

Described herein is a pharmaceutical composition comprising rasagiline mesylate having a D₉₀ particle size of 255 microns to 1500 microns, and one or more pharmaceutically acceptable excipients.

Also described herein is a pharmaceutical composition comprising rasagiline mesylate having a D₉₀ particle size of 255 microns to 1500 microns made by the process disclosed herein, and one or more pharmaceutically acceptable excipients.

Also encompassed is a process for preparing a pharmaceutical formulation comprising combining rasagiline mesylate has a D₉₀ particle size of 255 microns to 1500 microns with one or more pharmaceutically acceptable excipients.

Pharmaceutical compositions may comprise at least a therapeutically effective amount of rasagiline mesylate having a D₉₀ particle size of 255 microns to 1500 microns. Such pharmaceutical compositions may be administered to a mammalian patient in a dosage form, e.g., solid, liquid, powder, elixir, aerosol, syrups, injectable solution, etc. Dosage forms may be adapted for administration to the patient by oral, buccal, parenteral, ophthalmic, rectal and transdermal routes or any other acceptable route of administration. Oral dosage forms include, but are not limited to, tablets, pills, capsules, syrup, troches, sachets, suspensions, powders, lozenges, elixirs and the like. The rasagiline mesylate having a D₉₀ particle size of 255 microns to 1500 microns may also be administered as suppositories, ophthalmic ointments and suspensions, and parenteral suspensions, which are administered by other routes.

The pharmaceutical compositions further contain one or more pharmaceutically acceptable excipients. Suitable excipients and the amounts to use may be readily determined by the formulation scientist based upon experience and consideration of standard procedures and reference works in the field, e.g., the buffering agents, sweetening agents, binders, diluents, fillers, lubricants, wetting agents and disintegrants described hereinabove.

Capsule dosage forms may contain rasagiline mesylate having a D₉₀ particle size of 255 microns to 1500 microns within a capsule which may be coated with gelatin. Tablets and powders may also be coated with an enteric coating. Suitable enteric coating include phthalic acid cellulose acetate, hydroxypropylmethyl cellulose phthalate, polyvinyl alcohol phthalate, carboxy methyl ethyl cellulose, a copolymer of styrene and maleic acid, a copolymer of methacrylic acid and methyl methacrylate, and like materials, and if desired, the coating agents may be employed with suitable plasticizers and/or extending agents. A coated capsule or tablet may have a coating on the surface thereof or may be a capsule or tablet comprising a powder or granules with an enteric-coating.

Tableting compositions may have few or many components depending upon the tableting method used, the release rate desired and other factors. For example, the compositions described herein may contain diluents such as cellulose-derived materials like powdered cellulose, microcrystalline cellulose, microfine cellulose, methyl cellulose, ethyl cellulose, hydroxyethyl cellulose, hydroxypropyl cellulose, hydroxypropylmethyl cellulose, carboxymethyl cellulose salts and other substituted and unsubstituted celluloses; starch; pregelatinized starch; inorganic diluents such calcium carbonate and calcium diphosphate and other diluents known to one of ordinary skill in the art. Yet other suitable diluents include waxes, sugars (e.g. lactose) and sugar alcohols such as mannitol and sorbitol, acrylate polymers and copolymers, as well as pectin, dextrin and gelatin.

Other excipients include binders, such as acacia gum, pregelatinized starch, sodium alginate, glucose and other binders used in wet and dry granulation and direct compression tableting processes; disintegrants such as sodium starch glycolate, crospovidone, low-substituted hydroxypropyl cellulose and others; lubricants like magnesium and calcium stearate and sodium stearyl fumarate; flavorings; sweeteners; preservatives; pharmaceutically acceptable dyes and glidants such as silicon dioxide.

### INSTRUMENTAL DETAILS:

### Particle Size Method of Analysis:

Particle Size Distribution (PSD)" is determined by laser diffraction in a Malvern Master Sizer 2000 equipment or its equivalent:

**Measurement conditions:**

| | | |
|---|---|---|
| Dispersant | : | Sunflower oil (refractive Index: 1.469) |
| Sample preparation | : | Transfer about 250 mg of the sample in to a Petri dish; add few drops of sunflower oil (dispersant) to make a paste. Prepare the sample just before the addition to dispersant unit |
| | | |
| Calculation model | : | Fraunhufer |
| Calculation model | : | General purpose, normal sensitivity, irregular Particles |
| Obscuration limits | : | 10 - 20% |
| Obscuration filtering | : | On |
| Background measuring time | : | 30 seconds |
| Stirring speed | : | 2800 RPM |
| Number of samplings per sample | : | 1 |
| Number of measurements per sample | : | 6 |
| Delay between measurements | : | 10 seconds |

### Gas Chromatographic (GC) Method:

Residual Solvent Content was measured by Gas Chromatography (GC) by using the Agilent 6890N system equipped with a flame ionization detector and a Head space sampler (Head space G1888 or its equivalent) with Empower chromatography software or its equivalent under the following conditions:

### Column:

DB-624; 30 meter length, 0.32 mm internal diameter, 1.8 µm film thickness, fused silica capillary column Agilent make. P/No: 125-1334 or its equivalent.

**GC parameters:**

| | |
|---|---|
| Oven Temperature (1) | : 40°C |
| Time (1) | : 5 minutes |
| Rate (1) | : 2°C per minute |
| Oven Temperature (2) | : 60°C |
| Time (2) | : 0 minutes |
| Rate (2) | : 6°C per minute |
| Oven Temperature (3) | : 120°C |
| Time (3) | : 5 minutes |
| Rate (3) | : 20°C per minute |
| Oven Temperature (4) | : 200°C |
| Time (4) | : 5 minutes |
| Injector temperature | : 200°C |
| Detector temperature | : 250°C |
| Carrier gas, Helium flow | : 1.0 mL/minute |
| Split ratio | : 10:1 |
| Detector temperature | : 250°C |
| Hydrogen flow | : 30 mL/minute |
| Zero air flow | : 300 mL/minute |

The following examples are given for the purpose of illustrating the present disclosure.

### EXAMPLES

### Example 1

Rasagiline mesylate (5 g) was added to ethyl acetate (150 ml) and the mixture was heated to reflux, followed by the addition of methanol (20 ml), to form a clear solution, and then stirred for 15 minutes. The resulting solution was slowly cooled to 60°C with slow stirring. The stirring was stopped and the reaction mass was maintained for 1 hour at same temperature to grow the crystals. The resulting mass was further cooled to 45-50°C and kept for 12 hours at 45-50°C. The resulting mass was finally cooled to 25-30°C, the material was filtered and then dried under vacuum at 60 - 65°C to produce 3.7 g of rasagiline mesylate [Purity by HPLC: 99.92%; Particle size Data: (D₉₀) = 1407 microns; (D₅₀) = 663 microns].

Level of organic volatile impurities: methanol - 10 parts per million (ppm), isopropyl alcohol - 294 ppm, and toluene - 17 ppm.

### Example 2

Rasagiline base (10 g) was added to a mixture of ethyl acetate (300 ml) and methanol (13 ml), and the mixture was heated to 45-50°C. The resulting mass was followed by the addition of methane sulfonic acid (5.89 g) and the mass temperature was raised to reflux. Methanol (32 ml) was added to the resulting mass to provide a clear solution, followed by stirring for 15 minutes. The reaction mass was slowly cooled to 60°C without stirring and kept for 1 hour at 60°C. The resulting mass was further cooled to 50-55°C and kept for 12 hours at 50-55°C. The resulting mass was further cooled to 40-45°C and maintained for 1-2 hours at 40-45°C. The resulting mass was finally cooled to room temperature (25-30°C) and maintained for 2 hours. The resulting solid was filtered and then dried at 60 - 65°C to produce 9.7 g of rasagiline mesylate [Purity by HPLC: 99.95%; Particle size Data: (D₉₀)= 1496 microns; (D₅₀)= 864 microns].

Level of organic volatile impurities: methanol - 15 parts per million (ppm), isopropyl alcohol - 265 ppm, and toluene - 18 ppm.

### Reference Example 3

Rasagiline mesylate (obtained from examples 1-2) was fine-milled by being passed through a grinder (Make: Morphy Richards, Model-Icon DLX) having a stainless steel liquidizing blade for 3-4 minutes to obtain 90 volume-% of the rasagiline mesylate particles having a diameter of less than 300 microns.

### Reference Example 4

Rasagiline mesylate (710 g, obtained from example 2) was ground in a mixer (Make: Morphy Richards, Model-Icon DLX) having a stainless steel liquidizing blade for 3-4 minutes. The obtained powder was passed through a sieve (B.S.S.-100, A.S.T.M - 100, Micron - 500) to provide 90 volume-% of the rasagiline mesylate particles (660 g) having a diameter of less than about 400.

Unless otherwise indicated, the following definitions are set forth to illustrate and define the meaning and scope of the various terms used herein.

The term "pharmaceutically acceptable" means that which is useful in preparing a pharmaceutical composition that is generally non-toxic and is not biologically undesirable and includes that which is acceptable for veterinary use and/or human pharmaceutical use.

The term "pharmaceutical composition" is intended to encompass a drug product including the active ingredient(s), pharmaceutically acceptable excipients that make up the carrier, as well as any product which results, directly or indirectly, from combination, complexation or aggregation of any two or more of the ingredients. Accordingly, the pharmaceutical compositions encompass any composition made by admixing the active ingredient, active ingredient dispersion or composite, additional active ingredient(s), and pharmaceutically acceptable excipients.

The term "therapeutically effective amount" as used herein means the amount of a compound that, when administered to a mammal for treating a state, disorder or condition, is sufficient to effect such treatment. The "therapeutically effective amount" will vary depending on the compound, the disease and its severity and the age, weight, physical condition and responsiveness of the mammal to be treated.

The term "delivering" as used herein means providing a therapeutically effective amount of an active ingredient to a particular location within a host causing a therapeutically effective blood concentration of the active ingredient at the particular location. This can be accomplished, e.g., by topical, local or by systemic administration of the active ingredient to the host.

The term "buffering agent" as used herein is intended to mean a compound used to resist a change in pH upon dilution or addition of acid of alkali. Such compounds include, by way of example and without limitation, potassium metaphosphate, potassium phosphate, monobasic sodium acetate and sodium citrate anhydrous and dehydrate and other such material known to those of ordinary skill in the art.

The term "sweetening agent" as used herein is intended to mean a compound used to impart sweetness to a formulation. Such compounds include, by way of example and without limitation, aspartame, dextrose, glycerin, mannitol, saccharin sodium, sorbitol, sucrose, fructose and other such materials known to those of ordinary skill in the art.

The term "binders" as used herein is intended to mean substances used to cause adhesion of powder particles in granulations. Such compounds include, by way of example and without limitation, acacia, alginic acid, tragacanth, carboxymethylcellulose sodium, polyvinylpyrrolidone, compressible sugar (e.g., NuTab), ethylcellulose, gelatin, liquid glucose, methylcellulose, pregelatinized starch, starch, polyethylene glycol, guar gum, polysaccharide, bentonites, sugars, invert sugars, poloxamers (PLURONIC(™) F68, PLURONIC(™) F127), collagen, albumin, celluloses in non-aqueous solvents, polypropylene glycol, polyoxyethylene-polypropylene copolymer, polyethylene ester, polyethylene sorbitan ester, polyethylene oxide, microcrystalline cellulose, combinations thereof and other material known to those of ordinary skill in the art.

The term "diluent" or "filler" as used herein is intended to mean inert substances used as fillers to create the desired bulk, flow properties, and compression characteristics in the preparation of solid dosage formulations. Such compounds include, by way of example and without limitation, dibasic calcium phosphate, kaolin, sucrose, mannitol, microcrystalline cellulose, powdered cellulose, precipitated calcium carbonate, sorbitol, starch, combinations thereof and other such materials known to those of ordinary skill in the art.

The term "glidant" as used herein is intended to mean agents used in solid dosage formulations to improve flow-properties during tablet compression and to produce an anti-caking effect. Such compounds include, by way of example and without limitation, colloidal silica, calcium silicate, magnesium silicate, silicon hydrogel, cornstarch, talc, combinations thereof and other such materials known to those of ordinary skill in the art.

The term "lubricant" as used herein is intended to mean substances used in solid dosage formulations to reduce friction during compression of the solid dosage. Such compounds include, by way of example and without limitation, calcium stearate, magnesium stearate, mineral oil, stearic acid, zinc stearate, combinations thereof and other such materials known to those of ordinary skill in the art.

The term "disintegrant" as used herein is intended to mean a compound used in solid dosage formulations to promote the disruption of the solid mass into smaller particles which are more readily dispersed or dissolved. Exemplary disintegrants include, by way of example and without limitation, starches such as corn starch, potato starch, pregelatinized, sweeteners, clays, such as bentonite, microcrystalline cellulose (e.g., Avicel(™)), carsium (e.g., Amberlite(™)), alginates, sodium starch glycolate, gums such as agar, guar, locust bean, karaya, pectin, tragacanth, combinations thereof and other such materials known to those of ordinary skill in the art.

The term "wetting agent" as used herein is intended to mean a compound used to aid in attaining intimate contact between solid particles and liquids. Exemplary wetting agents include, by way of example and without limitation, gelatin, casein, lecithin (phosphatides), gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers (e.g., macrogol ethers such as cetomacrogol 1000), polyoxyethylene castor oil derivatives, polyoxyethylene sorbitan fatty acid esters, (e.g., TWEEN(™)s), polyethylene glycols, polyoxyethylene stearates colloidal silicon dioxide, phosphates, sodium dodecylsulfate, carboxymethylcellulose calcium, carboxymethylcellulose sodium, methylcellulose, hydroxyethylcellulose, hydroxyl propylcellulose, hydroxypropylmethylcellulose phthalate, noncrystalline cellulose, magnesium aluminum silicate, triethanolamine, polyvinyl alcohol, and polyvinylpyrrolidone (PVP). Tyloxapol (a nonionic liquid polymer of the alkyl aryl polyether alcohol type) is another useful wetting agent, combinations thereof and other such materials known to those of ordinary skill in the art.

The term "micronization" used herein means a process or method by which the size of a population of particles is reduced.

As used herein, the term "micron" or "µm" both are same refers to "micrometer" which is 1x10⁻⁶ meter.

As used herein, "crystalline particles" means any combination of single crystals, aggregates and agglomerates.

As used herein, "Particle Size Distribution (P.S.D)" means the cumulative volume size distribution of equivalent spherical diameters as determined by laser diffraction in Malvern Master Sizer 2000 equipment or its equivalent.

As used herein, Dx means that X percent of the particles have a diameter less than a specified diameter D. Thus, a D₉₀ or d(0.9) of less than 300 microns means that 90 volume-percent of the particles in a composition have a diameter less than 300 microns.

The important characteristics of the PSD were the (D₉₀), which is the size, in microns, below which 90% of the particles by volume are found, and the (D₅₀), which is the size, in microns, below which 50% of the particles by volume are found.

As used herein, "blend uniformity" refers to the homogeneity of granulate including rasagiline mesylate particles before tablet formulation, and can represent one sample or the average of more than one sample.

By "substantially pure" is meant having purity greater than about 99%, specifically greater than about 99.90%, and more specifically greater than about 99.95% as measured by HPLC.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. The term wt% refers to percent by weight. All methods described herein can be performed in any suitable order unless otherwise indicated herein or otherwise clearly contradicted by context. The use of any and all examples, or exemplary language (e.g., "such as") provided herein, is intended merely to better illuminate the disclosure.

## Claims

1. Rasagiline mesylate particles having a D₉₀ particle size of 600 microns to 1500 microns, wherein the rasagiline mesylate has a total purity of 99% to 99.99% as measured by HPLC.

2. The rasagiline mesylate of claim 1, having less than 200 parts per million (ppm) methanol, less than 500 ppm isopropyl alcohol, less than 100 ppm toluene, and less than 200 ppm ethyl acetate.

3. The rasagiline mesylate of claim 2, wherein the rasagiline mesylate has less than 25 ppm methanol, less than 350 ppm isopropyl alcohol, less than 30 ppm toluene, and less than 30 ppm ethyl acetate; and wherein the rasagiline mesylate has the overall level of organic volatile impurities in an amount of less than 500 ppm.

4. A process for the preparation of rasagiline mesylate having a D₉₀ particle size of 600 microns to 1500 microns of claim 1, comprising:
a) providing a solution of rasagiline mesylate in a solvent medium comprising an ester solvent and an alcoholic solvent;
b) subjecting the solution from step-(a) to gradual cooling to produce a cooled solution;
c) optionally, seeding the solution obtained in step-(b); and
d) crystallizing rasagiline mesylate particles having a D₉₀ particle size of 600 microns to 1500 microns from the cooled solution, wherein the rasagiline mesylate obtained has a total purity of 99% to 99.99% as measured by HPLC.

5. The process of claim 4, wherein the alcohol solvent is selected from the group consisting of methanol, ethanol, propanol, isopropanol, n-butanol, tert-butanol, amyl alcohol, isoamyl alcohol, hexanol, and mixtures thereof; wherein the ester solvent is selected from the group consisting of ethyl acetate, isopropyl acetate, n-butyl acetate, tert-butyl acetate, and mixtures thereof; and wherein the ester solvent in step-(a) is used in an amount of 2 to 12 volumes with respect to the alcohol solvent.

6. The process of claim 5, wherein the alcoholic solvent is methanol; wherein the ester solvent is ethyl acetate; and wherein the ester solvent is used in an amount of 4 to 10 volumes with respect to the alcohol solvent.

7. The process of claim 4, wherein the solution in step-(a) is prepared by dissolving rasagiline mesylate in the solvent medium comprising an ester solvent and an alcoholic solvent at a temperature of 70ºC to the reflux temperature of the solvent medium.

8. The process of claim 4, wherein the solution in step-(a) is prepared by suspending rasagiline mesylate in the ester solvent, stirring the suspension at reflux, followed by slow addition of the alcoholic solvent.

9. The process of claim 4, wherein the solution in step-(a) is prepared by admixing rasagiline base, methanesulfonic acid, and the solvent medium to obtain a mixture, and heating the mixture at a temperature of 70ºC to the reflux temperature of the solvent medium to provide the rasagiline mesylate solution.

10. The process of claim 4, wherein the solution in step-(a) is prepared by admixing rasagiline base, methanesulfonic acid, and the solvent medium to obtain a mixture, heating the mixture at a temperature of 70ºC to the reflux temperature of the solvent medium, and adding an alcoholic solvent to the resulting mixture to obtain the rasagiline mesylate solution.

11. The process of claim 4, wherein the gradual cooling of the solution in step-(b) is performed by slowly cooling the solution initially to a temperature of below 65ºC, maintaining the solution at the same temperature for at least 15 minutes, further cooling the solution to a temperature of below 50ºC, and maintaining the solution at the same temperature for at least 15 minutes; and wherein the crystallization in step-(d) is carried out by cooling the solution at a temperature of below 30ºC for at least 15 minutes.

12. The process of claim 11, wherein the gradual cooling of the solution is performed by slowly cooling the solution initially to a temperature of 55ºC to 65ºC for at least 15 minutes, maintaining the solution at the same temperature for 30 minutes to 3 hours, further cooling the solution to a temperature of 40ºC to 50ºC for at least 15 minutes, and maintaining the solution at the same temperature for 3 hours to 15 hours; and wherein the crystallization in step-(d) is carried out by cooling the solution at a temperature of 0ºC to 30ºC for 30 minutes to 20 hours.

13. The process of claim 4, wherein the rasagiline mesylate solid obtained in step-(d) is collected by filtration, filtration under vacuum, decantation, and centrifugation, filtration employing a filtration media of a silica gel or celite, or a combination thereof; wherein the rasagiline mesylate obtained in step-(d) is further dried under vacuum or at atmospheric pressure, at a temperature of 35ºC to 70ºC.

## Patentansprüche

1. Rasagilinmesylat-Partikel mit einer D₉₀-Partikelgröße von 600 Mikrometer bis 1500 Mikrometer, wobei das Rasagilinmesylat eine Gesamtreinheit von 99 % bis 99,99 %, wie durch HPLC gemessen, aufweist.

2. Rasagilinmesylat nach Anspruch 1, mit weniger als 200 Teilchen pro Million (ppm) Methanol, weniger als 500 ppm Isopropylalkohol, weniger als 100 ppm Toluol und weniger als 200 ppm Ethylacetat.

3. Rasagilinmesylat nach Anspruch 2, wobei das Rasagilinmesylat weniger als 25 ppm Methanol, weniger als 350 ppm Isopropylalkohol, weniger als 30 ppm Toluol und weniger als 30 ppm Ethylacetat aufweist; und wobei das Rasagilinmesylat den Gesamtgehalt von organischen flüchtigen Verunreinigungen in einer Menge von weniger als 500 ppm aufweist.

4. Vorgang zum Herstellen von Rasagilinmesylat mit einer D₉₀-Partikelgröße von 600 Mikrometer bis 1500 Mikrometer nach Anspruch 1, Folgendes umfassend:
a) Bereitstellen einer Lösung von Rasagilinmesylat in einem Lösungsmittelmedium, umfassend ein Ester-Lösungsmittel und ein alkoholisches Lösungsmittel;
b) Aussetzen der Lösung aus Schritt (a) einem allmählichen Kühlen zum Erzeugen einer gekühlten Lösung;
c) optional Impfen der in Schritt (b) erhaltenen Lösung; und
d) Auskristallisieren von Rasagilinmesylat-Partikeln mit einer D₉₀-Partikelgröße von 600 Mikrometer bis 1500 Mikrometer aus der gekühlten Lösung, wobei das erhaltene Rasagilinmesylat eine Gesamtreinheit von 99 % bis 99,99 %, wie durch HPLC gemessen, aufweist.

5. Vorgang nach Anspruch 4, wobei das Alkohol-Lösungsmittel ausgewählt ist aus der Gruppe bestehend aus Methanol, Ethanol, Propanol, Isopropanol, n-Butanol, tert-Butanol, Amylalkohol, Isoamylalkohol, Hexanol und Mischungen daraus; wobei das Ester-Lösungsmittel ausgewählt ist aus der Gruppe bestehend aus Ethylacetat, Isopropylacetat, n-Butylacetat, tert-Butylacetat und Mischungen daraus; und wobei das Ester-Lösungsmittel in Schritt (a) in einer Menge eines 2- bis 12-fachen Volumens bezogen auf das Alkohol-Lösungsmittel verwendet wird.

6. Vorgang nach Anspruch 5, wobei das alkoholische Lösungsmittel Methanol ist; wobei das Ester-Lösungsmittel Ethylacetat ist; und wobei das Ester-Lösungsmittel in einer Menge eines 4- bis 10-fachen Volumens bezogen auf das Alkohol-Lösungsmittel verwendet wird.

7. Vorgang nach Anspruch 4, wobei die Lösung in Schritt (a) durch Auflösen von Rasagilinmesylat in dem Lösungsmittelmedium, umfassend ein Ester-Lösungsmittel und ein alkoholisches Lösungsmittel, bei einer Temperatur von 70 °C bis zur Rückflusstemperatur des Lösungsmittelmediums hergestellt wird.

8. Vorgang nach Anspruch 4, wobei die Lösung in Schritt (a) durch Suspendieren von Rasagilinmesylat im Ester-Lösungsmittel, Rühren der Suspension unter Rückfluss, gefolgt von einem langsamen Zugeben des alkoholischen Lösungsmittels hergestellt wird.

9. Vorgang nach Anspruch 4, wobei die Lösung in Schritt (a) durch Beimengen von Rasagilinbasis, Methansulfonsäure und dem Lösungsmittelmedium zum Erhalten einer Mischung und durch Erwärmen der Mischung bei einer Temperatur von 70 °C auf die Rückflusstemperatur des Lösungsmittelmediums hergestellt wird, um die Rasagilinmesylatlösung bereitzustellen.

10. Vorgang nach Anspruch 4, wobei die Lösung in Schritt (a) durch Beimengen von Rasagilinbasis, Methansulfonsäure und dem Lösungsmittelmedium zum Erhalten einer Mischung, durch Erwärmen der Mischung bei einer Temperatur von 70 °C auf die Rückflusstemperatur des Lösungsmittelmediums und durch Hinzufügen eines alkoholischen Lösungsmittels zur resultierenden Mischung hergestellt wird, um die Rasagilinmesylatlösung zu erhalten.

11. Vorgang nach Anspruch 4, wobei das allmähliche Kühlen der Lösung in Schritt (b) durch Folgendes durchgeführt wird: langsames Kühlen der Lösung zunächst auf eine Temperatur unter 65 °C, Beibehalten der Lösung auf der gleichen Temperatur wenigstens 15 Minuten lang, ferner Kühlen der Lösung auf eine Temperatur unter 50 °C und Beibehalten der Lösung auf der gleichen Temperatur wenigstens 15 Minuten lang; und wobei das Auskristallisieren in Schritt (d) durch Kühlen der Lösung auf eine Temperatur von unter 30 °C wenigstens 15 Minuten lang ausgeführt wird.

12. Vorgang nach Anspruch 11, wobei das allmähliche Kühlen der Lösung durch Folgendes durchgeführt wird: langsames Kühlen der Lösung zunächst auf eine Temperatur von 55 °C bis 65 °C wenigstens 15 Minuten lang, Beibehalten der Lösung auf der gleichen Temperatur 30 Minuten bis 3 Stunden lang, weiteres Kühlen der Lösung auf eine Temperatur von 40 °C bis 50 °C wenigstens 15 Minuten lang und Beibehalten der Lösung auf der gleichen Temperatur 3 Stunden bis 15 Stunden lang; und wobei das Auskristallisieren in Schritt (d) durch Kühlen der Lösung bei einer Temperatur von 0 °C bis 30 °C 30 Minuten bis 20 Stunden lang ausgeführt wird.

13. Vorgang nach Anspruch 4, wobei der in Schritt (d) erhaltene Rasagilinmesylat-Feststoff durch Filtrieren, Vakuumfiltrieren, Dekantieren und Zentrifugieren gesammelt wird, wobei das Filtrieren ein Filtermedium aus einem Kieselsäuregel oder einem Celit oder einer Kombination daraus einsetzt; wobei das in Schritt (d) erhaltene Rasagilinmesylat ferner unter Vakuum oder bei Atmosphärendruck bei einer Temperatur von 35 °C bis 70 °C getrocknet wird.

## Revendications

1. Particules de mésylate de rasagiline ayant une taille de particule D₉₀ de 600 microns à 1 500 microns, dans lesquelles le mésylate de rasagiline a une pureté totale de 99 % à 99,99 % telle que mesurée par HPLC.

2. Mésylate de rasagiline selon la revendication 1, ayant moins de 200 parties par million (ppm) de méthanol, moins de 500 ppm d'alcool isopropylique, moins de 100 ppm de toluène, et moins de 200 ppm d'acétate d'éthyle.

3. Mésylate de rasagiline selon la revendication 2, dans lequel le mésylate de rasagiline a moins de 25 ppm de méthanol, moins de 350 ppm d'alcool isopropylique, moins de 30 ppm de toluène, et moins de 30 ppm d'acétate d'éthyle ; et dans lequel le mésylate de rasagiline a un taux global d'impuretés volatiles organiques dans une quantité de moins de 500 ppm.

4. Procédé de préparation de mésylate de rasagiline ayant une taille de particule D₉₀ de 600 microns à 1 500 microns de la revendication 1, comprenant :
a) la fourniture d'une solution de mésylate de rasagiline dans un milieu solvant comprenant un solvant ester et un solvant alcoolique ;
b) la soumission de la solution de l'étape (a) à un refroidissement progressif pour produire une solution refroidie ;
c) facultativement, l'ensemencement de la solution obtenue à l'étape (b) ; et
d) la cristallisation de particules de mésylate de rasagiline ayant une taille de particule D₉₀ de 600 microns à 1 500 microns à partir de la solution refroidie, le mésylate de rasagiline obtenu ayant une pureté totale de 99 % à 99,99 % telle que mesurée par HPLC.

5. Procédé selon la revendication 4, dans lequel le solvant alcool est choisi dans le groupe consistant en le méthanol, l'éthanol, le propanol, l'isopropanol, le n-butanol, le tert-butanol, l'alcool amylique, l'alcool isoamylique, l'hexanol, et leurs mélanges ; dans lequel le solvant ester est choisi dans le groupe consistant en l'acétate d'éthyle, l'acétate d'isopropyle, l'acétate de n-butyle, l'acétate de tert-butyle, et leurs mélanges ; et dans lequel le solvant ester à l'étape (a) est utilisé dans une quantité de 2 à 12 volumes par rapport au solvant alcool.

6. Procédé selon la revendication 5, dans lequel le solvant alcoolique est le méthanol ; dans lequel le solvant ester est l'acétate d'éthyle ; et dans lequel le solvant ester est utilisé dans une quantité de 4 à 10 volumes par rapport au solvant alcool.

7. Procédé selon la revendication 4, dans lequel la solution à l'étape (a) est préparée par dissolution du mésylate de rasagiline dans le milieu solvant comprenant un solvant ester et un solvant alcoolique à une température de 70 °C jusqu'à la température de reflux du milieu solvant.

8. Procédé selon la revendication 4, dans lequel la solution à l'étape (a) est préparée par mise en suspension du mésylate de rasagiline dans le solvant ester, agitation de la suspension au reflux, suivie par l'addition lente du solvant alcoolique.

9. Procédé selon la revendication 4, dans lequel la solution à l'étape (a) est préparée par mélange de rasagiline base, d'acide méthanesulfonique, et du milieu solvant pour obtenir un mélange, et chauffage du mélange à une température de 70 °C jusqu'à la température de reflux du milieu solvant pour fournir la solution de mésylate de rasagiline.

10. Procédé selon la revendication 4, dans lequel la solution à l'étape (a) est préparée par mélange de rasagiline base, d'acide méthanesulfonique, et du milieu solvant pour obtenir un mélange, chauffage du mélange à une température de 70 °C jusqu'à la température de reflux du milieu solvant, et addition d'un solvant alcoolique au mélange résultant pour obtenir la solution de mésylate de rasagiline.

11. Procédé selon la revendication 4, dans lequel le refroidissement progressif de la solution à l'étape (b) est effectué par refroidissement lent de la solution initialement à une température de moins de 65 °C, maintien de la solution à la même température pendant au moins 15 minutes, refroidissement supplémentaire de la solution à une température de moins de 50 °C, et maintien de la solution à la même température pendant au moins 15 minutes ; et dans lequel la cristallisation à l'étape (d) est réalisée par refroidissement de la solution à une température de moins de 30 °C pendant au moins 15 minutes.

12. Procédé selon la revendication 11, dans lequel le refroidissement progressif de la solution est effectué par refroidissement lent de la solution initialement à une température de 55 °C à 65 °C pendant au moins 15 minutes, maintien de la solution à la même température pendant 30 minutes à 3 heures, refroidissement supplémentaire de la solution à une température de 40 °C à 50 °C pendant au moins 15 minutes, et maintien de la solution à la même température pendant 3 heures à 15 heures ; et dans lequel la cristallisation à l'étape (d) est réalisée par refroidissement de la solution à une température de 0 °C à 30 °C pendant 30 minutes à 20 heures.

13. Procédé selon la revendication 4, dans lequel le solide de mésylate de rasagiline obtenu à l'étape (d) est collecté par filtration, filtration sous vide, transvasement, et centrifugation, filtration employant des milieux de filtration d'un gel de silice ou de célite, ou une de leurs combinaisons ; dans lequel le mésylate de rasagiline obtenu à l'étape (d) est en outre séché sous vide ou à la pression atmosphérique, à une température de 35 °C à 70 °C.
